# EUROPÄISCHE PATENTANMELDUNG

(11) **EP 0 696 640 A2**
(43) Veröffentlichungstag der Anmeldung: **14.02.1996**
(21) Anmeldenummer: 95112700.0
(22) Anmeldetag: 11.08.1995
(51) Int. Cl.: C12N 15/40, C07K 14/18, G01N 33/569, A61K 39/29

(54) **Rekombinantes Antigen aus der NS3-Region des Hepatitis C Virus**

(30) Priorität: 12.08.1994 DE 4428705
(71) Anmelder: BOEHRINGER MANNHEIM GMBH, D-68305 Mannheim (DE)
(72) Erfinder: Seidel, Christoph, Dr., D-82362 Weilheim (DE); Wienhüs, Ursula-Henrike, Dr., D-82152 Krailing (DE); Schmitt, Urban, D-82386 Oberhausen (DE); Motz, Manfred, Dr, D-80689 München (DE); Wiedmann, Michael, Dr., D-82377 Penzberg (DE); Upmeier, Barbara, Dr., D-82393 Iffeldorf (DE); Soutschek, Erwin, Dr., D-82335 Berg (DE)
(74) Vertreter: Weiss, Wolfgang, Dr. Dipl.-Chem.

(57) **Zusammenfassung**

Die vorliegende Erfindung betrifft ein Polypeptid, das aus den Aminosäuren 1207 ± 10 bis 1488 ± 10 eines Hepatitis C Virus und weniger als 20 fremden Aminosäuren besteht und die Verwendung dieses Polypeptids als Antigen in einem immunologischen Test.

## Beschreibung

Die Erfindung betrifft Polypeptide aus der Nicht-Strukturprotein 3 (NS3)-Region des Hepatitis C-Virus (HCV), eine für derartige Polypeptide kodierende Nucleinsäure sowie die Verwendung der Polypeptide als Antigen in einem immunologischen Testverfahren oder als Helicaseprotein.

Die als Non-A-non-B-Hepatitis bezeichnete Krankheit wird in vielen Fällen durch das Hepatitis C-Virus (HCV) hervorgerufen. Bei HCV handelt es sich um ein einzelsträngiges verkapseltes RNA-Virus, dessen Genom aus etwa 9 bis 10.000 Basen besteht. Von diesem Genom werden Strukturproteine (Kern- und Hüllproteine) sowie Nicht-Strukturproteine kodiert. Die Nicht-Strukturprotein 3 (NS3)-Region von HCV enthält eine Protease und eine Helicase. Die Protease-Aktivität ist im aminoterminalen Drittel der NS3-Region lokalisiert.

In der europäischen Patentanmeldung EP-A-0 318 216 ist eine partielle Nucleotidsequenz von HCV offenbart. Es wird die Verwendung von Nucleinsäurefragmenten oder Polypeptidabschnitten aus HCV zum diagnostischen Nachweis sowie zur therapeutischen Behandlung beansprucht.

EP-A-0 450 931 offenbart die vollständige Nucleotid- und Aminosäuresequenz von HCV. Weiterhin wird eine Kombination von synthetischen HCV-Antigenen beschrieben, umfassend ein erstes HCV-Antigen aus der C-Domäne und mindestens ein weiteres HCV-Antigen aus einer der Nicht-Strukturdomänen NS3, NS4 oder NS5 und der Hülldomäne S. Ein bevorzugtes Antigen aus der Domäne NS3 ist ein mit C33c bezeichnetes Antigen, welches die Aminosäuren 1192 bis 1457 des in Figur 1 von EP-A-0 450 931 dargestellten HCV-Genoms umfaßt.

Die internationale Patentanmeldung WO92/11370 betrifft die Klonierung und Sequenzierung von verschiedenen Polypeptiden aus dem Genom von HCV und die Verwendung dieser Polypeptide ohne Fremdproteinanteile bei Testkits und als Impfstoff. Ein in Zusammenhang mit dieser Anmeldung bei der Deutschen Sammlung für Mikroorganismen und Zellkulturen GmbH, Mascheroder Weg 1b, 38124 Braunschweig, BRD (DSM) unter der Nummer 6847 hinterlegter Klon NS-3 enthält die genetische Information für ein Polypeptid von 527 Aminosäuren aus der NS3-Region von HCV.

Mori et al. (Jpn. J. Cancer Res. 83 (1992), 264-268) beschreiben den diagnostischen Nachweis einer HCV-Infektion durch Bestimmung viraler Antikörper in Blut unter Verwendung von viralen Proteinen als Antigene. Diese HCV-Proteine werden als Fusionsprotein mit β-Galactosidase in E.coli exprimiert. Aus der NS3-Region zeigte ein Protein, welches die Aminosäuren 1295 bis 1541 des HCV-Genoms enthielt, die höchste Sensitivität. Nachteilig bei derartigen Fusionsproteinen ist jedoch, daß Kreuzreaktionen mit dem anfusionierten Proteinanteil auftreten können, welche die Spezifität der Nachweisreaktion verringern.

Beim Nachweis von HCV in Blut ist eine hohe Spezifität und Sensitivität des Tests erforderlich. Weiterhin sollte das für den Test verwendete Antigen in hoher Ausbeute exprimierbar und stabil sein. Bisher bekannte Antigene aus dem HCV-Genom weisen Nachteile auf, da sie eine oder mehrere der obigen Anforderungen nicht erfüllen.

Eine der vorliegenden Erfindung zugrundeliegende Aufgabe bestand somit in der Bereitstellung eines Polypeptids aus dem HCV-Genom, bei dem diese Nachteile des Standes der Technik mindestens teilweise beseitigt sind und das insbesondere verglichen mit bekannten Antigenen eine höhere Spezifität und Sensitivität besitzt sowie in hoher Ausbeute exprimierbar und stabil ist.

Diese Aufgabe wird gelöst durch ein Polypeptid, das aus den Aminosäuren 1207 ± 10 bis 1488 ± 10 eines Hepatitis C-Virus und weniger als 20, vorzugsweise weniger als 15 fremden Aminosäuren besteht. Das erfindungsgemäße Polypeptid enthält vorzugsweise die Aminosäuren 1207 ± 5 bis 1488 ± 5, besonders bevorzugt 1207 ± 2 bis 1488 ± 2 und am meisten bevorzugt 1207 bis 1488 eines Hepatitis C-Virus, wobei sich die Numerierung der Aminosäurereste auf Fig. 1 von EP-A-0 450 931 bezieht.

Das erfindungsgemäße Polypeptid kann aus einem beliebigen HCV-Isolat stammen, beispielsweise aus einem HCV-Isolat mit einer Nucleotidsequenz, wie sie in EP-A-0 450 931 beschrieben ist. Vorzugsweise stammt jedoch das Polypeptid aus dem HCV-Isolat, aus dem der in WO92/11370 beschriebene Klon NS3 stammt, der bei der Deutschen Sammlung von Mikroorganismen und Zellkulturen GmbH (DSM), Mascheroder Weg 1b, 38124 Braunschweig, unter der Nummer DSM 6847 hinterlegt wurde. Besonders bevorzugt ist das erfindungsgemäße Polypeptid durch rekombinante Expression des Vektors pUC-D26 erhältlich.

In SEQ ID NO. 1 ist die Aminosäuresequenz eines erfindungsgemäßen Polypeptids gezeigt. Die Aminosäuren 1 - 13 sind fremde Amionosäuren. Die Aminosäuren 14 - 295 stammen aus HCV. Besonders bevorzugt enthält das erfindungsgemäße Polypeptid die Aminosäuren 14 - 295 der in SEQ ID NO. 1 und 2 dargestellten Aminosäuresequenz oder eine dazu mindestens 90% homologe Aminosäuresequenz.

Ein weiterer Gegenstand der vorliegenden Erfindung ist auch ein wie oben definiertes Polypeptid, das mindestens eine Markierungsgruppe trägt. Als Markierungsgruppe kommen alle bekannten Markierungsgruppen in Frage, die in einem Testsystem nachgewiesen werden können, d.h. direkt oder indirekt nachweisbare Markierungsgruppen. Dabei ist unter einer direkt nachweisbaren Markierungsgruppe eine solche Gruppe zu verstehen, die ein direkt nachweisbares Signal erzeugt, z.B. eine radioaktive Gruppe, eine Enzymgruppe, eine Lumineszenzgruppe, ein Metallkomplex etc.. Andererseits kann die Markierungsgruppe auch eine indirekt nachweisbare Gruppe sein, z.B. eine Biotin- oder Hapten-Gruppe, die durch Reaktion mit einem geeigneten Bindepartner (Streptavidin, Avidin bzw. Anti-Hapten-Antikörper), der wiederum eine Signal-erzeugende Gruppe trägt, nachweisbar ist. Die Markierungsgruppe kann mit dem Antigen auf bekannte Weise gekoppelt werden, beispielsweise über einen bifunktionellen Spacer. Derartige Verfahren zur Kopplung von Markierungsgruppen an Peptidantigene sind einem Fachmann auf dem Gebiet der Immunologie bekannt und brauchen hier nicht näher beschrieben werden.

Das erfindungsgemäße Polypeptid enthält vorzugsweise 1 bis 12 und besonders bevorzugt 3 bis 7 Markierungsgruppen.

Noch ein weiterer Gegenstand der Erfindung ist ein wie oben definiertes Polypeptid, bei dem eine oder mehrere der aus Cysteinresten stammenden Sulfhydrylgruppen in kovalent modifizierter Form vorliegen. Beispiele für geeignete kovalente Modifizierungsgruppen sind Maleimidodioxaoctylamin (MADOO), N-Methyl-Maleinimid (NMM), Jodessigsäure und Jodacetamid. Durch die kovalente Cysteinmodifikation wird eine besonders hohe spezifische immunologische Reaktivität erzielt.

Besonders bevorzugt erfolgt die kovalente Kopplung von direkt oder indirekt nachweisbaren Markierungsgruppen an die Sulfhydrylgruppen des Polypeptids. Beispiele für SH-reaktive bifunktionelle Linker zur Kopplung an Sulfhydrylgruppen sind Maleimidopropylamin (MP), Maleimidoethylamin (MEA) und Maleimidodioxaoctylamin (MADOO).

Weiterhin kann es bevorzugt sein, ein erfindungsgemäßes Polypeptid zu verwenden, bei dem ein oder mehrere Cysteinreste durch andere natürliche oder artifizielle Aminosäuren ersetzt sind. Vorzugsweise werden Cysteinreste durch strukturell analoge α-Aminosäuren, z.B. Serin oder α-Aminobuttersäure ersetzt. Durch Cysteinsubstitutionen wird eine besonders hohe Stabilität erhalten.

Das erfindungsgemäße Polypeptid weist gegenüber bereits bekannten Polypeptiden überraschende Vorteile auf. Gegenüber dem in EP-A-0 450 931 beschriebenen Antigen C33, welches die Aminosäurereste 1192 bis 1457 der HCV-Sequenz enthält, zeigt das erfindungsgemäße Polypeptid eine deutlich höhere Spezifität, die sich in einer statistisch signifikant geringeren Anzahl an falsch positiven Resultaten bei negativen Seren zeigt. Gegenüber dem in WO92/11370 beschriebenen Antigen NS3, welches den Bereich der Aminosäuren 1007 bis 1534 der HCV-Sequenz enthält, zeigt das erfindungsgemäße Polypeptid eine deutlich höhere Stabilität unter Testbedingungen. Gegenüber einem Polypeptid, das die Aminosäuren 1227 bis 1528 aus der NS3-Region von HCV enthält, hat das erfindungsgemäße Polypeptid den Vorteil einer besseren Expressionseffizienz und einer höheren Sensitivität. Aufgrund dieser Vorteile ist das erfindungsgemäße Polypeptid allen bisher bekannten HCV-Antigenen aus der NS3-Region deutlich überlegen.

Weiterhin betrifft die vorliegende Erfindung eine Nucleinsäure, die für ein erfindungsgemäßes Polypeptid kodiert. Ein bevorzugtes Beispiel für eine derartige Nucleinsäure ist die Fremd-DNA-Insertion im Vektor pUC-D26.

In SEQ ID NO.1 ist auch die Nucleotidsequenz einer erfindungsgemäßen Nucleinsäure gezeigt, die für das Polypeptid von SEQ ID NO. 1 und 2 kodiert. Die Nucleotide 40 - 885 kodieren für den aus HCV stammenden Bereich des Polypeptids. Die erfindungsgemäße Nucleinsäure enthält vorzugsweise (a) die Nucleotide 40 - 885 der in SEQ ID NO. 1 dargestellte Nucleotidsequenz oder (b) eine der Sequenz aus (a) im Rahmen der Degeneration des genetischen Codes entsprechende Nucleotidsequenz.

Ein weiterer Gegenstand der vorliegenden Erfindung ist ein Vektor, der mindestens eine Kopie einer erfindungsgemäßen Nucleinsäure enthält. Der erfindungsgemäße Vektor ist vorzugsweise ein prokaryontischer Vektor, d.h. ein zur Propagierung in einer prokaryontischen Wirtszelle geeigneter Vektor. Beispiele für solche Vektoren sind bei Sambrook et al. (Molecular Cloning. A Laboratory Manual, 2nd Ed., Cold Spring Harbor Laboratory Press (1989)), inbesondere in den Kapiteln 1 bis 4 und 17 dargestellt. Besonders bevorzugt ist der erfindungsgemäße Vektor ein zirkuläres Plasmid. Die erfindungsgemäße Nucleinsäure befindet sich auf dem Vektor vorzugsweise unter Kontrolle einer Promotorsequenz, welche die Expression des erfindungsgemäßen Polypeptids erlaubt. Ein bevorzugtes Beispiel für einen erfindungsgemäßen Vektor ist pUC-D26.

Weiterhin betrifft die vorliegende Erfindung eine Zelle, die mit mindestens einer Kopie einer erfindungsgemäßen Nucleinsäure oder eines erfindungsgemäßen Vektors transformiert ist. Vorzugsweise ist die Zelle eine prokaryontische Zelle, besonders bevorzugt eine gram-negative prokaryontische Zelle und am meisten bevorzugt eine E.coli-Zelle.

Das erfindungsgemäße Polypeptid wird vorzugsweise als Antigen in einem immunologischen Testverfahren verwendet. Anderseits kann das Polypeptid jedoch auch als Helicase-Protein und aufgrund seiner hervorragenden antigenen Wirkung zur Herstellung eines Impfstoffs gegen eine HCV-Infektion verwendet werden.

Außerdem betrifft die vorliegende Erfindung ein Verfahren zur immunologischen Bestimmung eines gegen Hepatitis C-Virus gerichteten Antikörpers in einer Probeflüssigkeit, welches dadurch gekennzeichnet ist, daß man die Probeflüssigkeit mit mindestens einem erfindungsgemäßen Polypeptid inkubiert und den Antikörper über eine Bindung mit dem Polypeptid nachweist. Dieses immunologische Bestimmungsverfahren kann an sich nach jedem bekannten Testformat erfolgen, z.B. in einem homogenen Immunoassay mit einer einzigen Reaktionsphase oder in einem heterogenen Immunoassay mit mehr als einer Reaktionsphase. Vorzugsweise wird ein heterogenes Testformat verwendet, bei dem das Vorhandensein des Antikörpers in Anwesenheit einer reaktiven Festphase nachgewiesen wird.

Eine Ausführungsform dieses Testformats ist das sogenannte Doppelantigen-Brückentestkonzept. Bei einem derartigen Verfahren inkubiert man die Probeflüssigkeit mit mindestens zwei erfindungsgemäßen Polypeptiden P₁ und P₂, wobei das Polypeptid P₁ (a) an eine Festphase gebunden ist oder (b) in einer an eine Festphase bindefähigen Form vorliegt, und das Polypeptid P₂ eine Markierungsgruppe trägt. Der Antikörper in der Probeflüssigkeit wird durch Bestimmung der Markierung in der Festphase oder/und in der flüssigen Phase, vorzugsweise in der Festphase, über einen immobilisierten, d.h. an die Festphase gebundenen Komplex nachgewiesen.

Die Testdurchführung beinhaltet vorzugsweise ein Mischen der Probeflüssigkeit mit einem gereinigten markierten Antigen P₂ sowie mit dem gereinigten festphasenseitigen Antigen P₁, um einen markierten immobilisierten Komplex aus markiertem Antigen, Antikörper und Festphasen-gebundenem Antigen zu erhalten. Gegenüber anderen Testformaten zum Nachweis von Antikörpern führen die Brückentestformate sowohl zu einer Verbesserung der Sensitivität, d.h. es werden zusätzliche Immunglobulinklassen wie etwa IgM erkannt, als auch der Spezifität, d.h. unspezifische Reaktivitäten mit Anti-IgG-Konjugat werden verringert.

Das markierte Antigen P₂ trägt eine direkt oder indirekt nachweisbare Markierungsgruppe wie oben beschrieben. Das festphasenseitige Antigen P₁ kann z.B. über einen bifunktionellen Spacer direkt an die Festphase gebunden sein. Vorzugsweise ist P₁ jedoch ein in der flüssigen Phase vorliegendes Konjugat aus einem erfindungsgemäßen Polypeptid und einem Reaktionspartner eines spezifischen Bindungssystems. Der andere Reaktionspartner des spezifischen Bindungssystems liegt an der Festphase gebunden vor. Beispiele für solche spezifischen Bindungssysteme sind Biotin/Avidin, Biotin/Streptavidin, Biotin/Antibiotin, Hapten/Antihapten, Kohlehydrat/Lectin und Antikörper bzw. Antikörperfragment und Antikörper gegen diesen Antikörper bzw. gegen das Antikörperfragment. Vorzugsweise liegt das Antigen P₁ in Form eines Biotin-Konjugats vor.

Das erfindungsgemäße Polypeptid-Antigen wird in einem derartigen Doppelantigen-Brückentest vorzugsweise in löslicher Form eingesetzt, um Leerwerterhöhungen und eine ungünstige Signal/Rausch-Relation aufgrund von Aggregationen des Antigens zu vermeiden. Hierzu wird das Antigen entweder in geeigneten Expressionssystemen bereits löslich exprimiert, oder nach Expression in löslicher Form auf bekannte Weise in vitro renaturiert. Weiterhin kann zur Vermeidung der Ausbildung von kovalent vernetzten Molekülaggregaten der immunologische Test unter milden reduzierenden Bedingungen (Zusatz von milden reduzierenden Reagenzien, vorzugsweise von Sulfhydrylreagenzien, bevorzugt von DTT (Dithiothreitol) oder DTE (Dithioerythritol) im Konzentrationsbereich von 1 mmol/l bis 25 mmol/l) durchgeführt oder/und bevorzugt ein an Sulfhydrylgruppen kovalent modifiziertes Antigen oder/und bevorzugt ein Antigen mit mindestens partiell substituierten Cysteinresten verwendet werden.

Das erfindungsgemäße Polypeptid-Antigen wird in einem derartigen Doppelantigen-Brückentest vorzugsweise in löslicher Form eingesetzt, um Leerwerterhöhungen und eine ungünstige Signal/Rausch-Relation aufgrund von Aggregationen des Antigens zu vermeiden. Eine ausführliche Beschreibung des Brückentestformats findet sich in EP-A-0 280 211. Auf diese Offenbarung wird hiermit Bezug genommen.

Das erfindungsgemäße Polypeptid kann aber auch in anderen Testformaten eingesetzt werden. Ein Beispiel hierfür ist ein indirekter Immunoassay zur Erkennung von spezifischem Immunglobulin durch Bindung an ein wandseitiges spezifisches Antigen und indirekten Nachweis über ein Konjugat mit einem zweiten Antikörper. Bei dieser Ausführungsform des erfindungsgemäßen Verfahrens inkubiert man die Probeflüssigkeit mit einem Polypeptid P₁, das (a) an eine Festphase gebunden ist oder (b) in einer an eine Festphase bindefähigen Form vorliegt, und mit einem weiteren, gegen P₁ gerichteten Antikörper, der eine Markierungsgruppe trägt. Der zu bestimmende Antikörper wird indirekt durch Bestimmung der Markierung in der Festphase oder/und in der flüssigen Phase, vorzugsweise in der Festphase nachgewiesen. Bei diesem Verfahren ist das an der Festphase durch einen immobilisierten Komplex aus markiertem Antikörper und festphasengebundenem Antigen erzeugte Signal indirekt proportional zur Konzentration von zu bestimmenden Antikörpern in der Probeflüssigkeit.

Ein weiterer Gegenstand der vorliegenden Erfindung ist ein Reagenz zur immunologischen Bestimmung eines gegen Hepatitis C-Virus gerichteten Antikörpers, das mindestens ein erfindungsgemäßes Polypeptid enthält. Wird das Reagenz in einem Doppelantigen-Brückentest verwendet, so enthält es vorzugsweise mindestens zwei Polypeptide P₁ und P₂, wobei das Polypeptid P₁ (a) an eine Festphase gebunden ist oder (b) in einer an eine Festphase bindefähigen Form vorliegt und das Polypeptid P₂ eine Markierungsgruppe trägt. Die Bindung des Polypeptids P₁ an die Festphase kann entweder durch direkte Bindung oder über ein spezifisches Bindepaar, vorzugsweise Streptavidin/Avidin und Biotin, erfolgen. Besonders bevorzugt liegt das Polypeptid P₁ in biotinylierter Form vor.

Bei Verwendung in einem indirekten Immuntest enthält das erfindungsgemäße Reagenz vorzugsweise ein Polypeptid P₁, das (a) an eine Festphase gebunden ist, oder (b) in einer an eine Festphase bindefähigen Form vorliegt, und einen gegen P₁ gerichteten Antikörper, der eine Markierungsgruppe trägt. Die Herstellung von gegen das erfindungsgemäße Polypeptid gerichteten Antikörpern erfolgt auf bekannte Weise durch Immunisierung von Versuchstieren mit dem entsprechenden Antigen und Gewinnung von polyklonalen Antiseren aus dem Versuchstier. Alternativ kann nach dem Verfahren von Köhler und Milstein oder einer Weiterentwicklung dieses Verfahrens ein monoklonaler Antikörper gegen das Antigen hergestellt werden. Anstelle eines vollständigen Antikörpers können auch Antikörperfragmente oder Antikörperderivate eingesetzt werden.

Ein weiteres Anwendungsgebiet der erfindungsgemäßen Polypeptide ist die Herstellung von Impfstoffen. Hierzu werden die erfindungsgemäßen Polypeptide vorzugsweise in gereinigter Form hergestellt und dann in Form von injizierbaren Flüssigkeiten gebracht, wobei es sich entweder um Lösungen oder Suspensionen der Polypeptide handeln kann. Die Polypeptide können auch in Liposomen eingeschlossen sein. Weitere Bestandteile der Impfstoffe sind z.B. Wasser, Salzlösungen, Glucose oder Glycerin. Darüber hinaus enthalten die Impfstoffe geringe Mengen an Hilfssubstanzen, wie Emulgatoren, Puffersubstanzen, gegebenenfalls Adjuvantien, die die Immunantwort steigern. Die Impfstoffe werden üblicherweise parenteral durch Injektion, vorzugsweise subkutan oder intramuskulär appliziert.

Noch ein weiterer Gegenstand der vorliegenden Erfindung ist ein Verfahren zur immunologischen Bestimmung eines Hepatitis C Virus gerichteten Antikörpers in einer Probeflüssigkeit, wobei man die Probeflüssigkeit mit mindestens einem Polypeptid, das Sequenzbereiche aus dem Hepatitis C Virus, insbesondere aus der NS3-Region von Hepatitis C Virus enthält, inkubiert und den Antikörper über eine Bindung mit dem Polypeptid nachweist, dadurch gekennzeichnet, daß man ein Polypeptid aus einem Bereich, der mindestens einen Cysteinrest enthält, verwendet und (a) die Bestimmung des Antikörpers unter reduzierenden Bedingungen durchführt, (b) einen oder mehrere Cysteinreste kovalent modifiziert oder/und (c) einen oder mehrere Cysteinreste durch andere Aminosäuren ersetzt.

Im Gegensatz zu anderen antigenen Bereichen aus HCV enthält die NS3-Region eine besonders große Häufung von Cysteinresten. Obwohl das im Verlauf der Virusvermehrung intrazellulär synthetisierte NS3-Protein stabil ist, erwies sich die Verwendung von NS3-Antigenen unter physiologischen Pufferbedingungen, z.B. in immunologischen Testverfahren, als äußerst problematisch, da unter diesen Bedingungen die freien Sulfhydrylgruppen der Cysteinreste leicht oxidieren. Dies führt sowohl zu intra- als auch zu intermolekularen Verletzungen des Antigens, wodurch dessen immunologische Reaktivität deutlich verringert wird.

Durch ein Testformat, bei dem ein oder mehrere NS3-Antigene mit modifizierten Cysteinresten oder/und mit substituierten Cysteinresten eingesetzt werden oder bei dem mild reduzierende Bedingungen, z.B. durch Zusatz eines Sulfhydrylreaenz, vorliegen, konnte nun überraschenderweise die immunologische Reaktivität dieser NS3-Antigene deutlich verbessert werden kann. Auf diese Weise wird sowohl eine frühzeitige Erkennung der Serokonversion als auch eine deutliche Verstärkung des Meßsignals erreicht.

Weiterhin wird die vorliegende Erfindung durch die nachfolgenden Beispiele und Sequenzprotokolle beschrieben.

Es zeigen:
- SEQ ID NO 1:: die Nucleotidsequenz eines bevorzugten erfindungsgemäßen Polypeptids,
- SEQ ID NO 2:: die Aminosäuresequenz einer bevorzugten erfindungsgemäßen Nucleinsäure,
- SEQ ID NO 3:: die Nucleotidsequenz von Primer (1)
- SEQ ID NO 4:: die Nucleotidsequenz von Primer (2)
- SEQ ID NO 5:: die Nucleotidsequenz von Primer (3)
- SEQ ID NO 6:: die Nucleotidsequenz von Primer (4)
- SEQ ID NO 7:: die Nucleotidsequenz von Primer (5) und
- SEQ ID NO 8:: die Nucleotidsequenz von Primer (6).

### BEISPIELE

### Beispiel 1

Klonierung und Expression eines Polypeptids mit den Aminosäuren 1207 bis 1488 aus dem NS3-Bereich des Hepatitis C-Virus

Mittels PCR wurde ausgehend von dem Klon NS3 (DSM 6847) unter Verwendung der Primer (1) und (2), deren Nucleotidsequenz in SEQ ID NO. 3 und SEQ ID NO. 4 gezeigt ist, ein DNA-Fragment amplifiziert. Am 5'-Ende dieses DNA-Fragments befinden sich Sequenzen für die Klonierung (BamHI-, BspHI-, EcoRI-Restriktionsschnittstellen) sowie ein ATG-Kodon und ein AAA(Lys)-Kodon zur Steigerung der Expression. Am 3'-Ende befinden sich Restriktionsstellen für HindIII und EcoRI sowie ein Stoppkodon (TTA). Der zu HCV homologe Bereich beginnt in den Primern (1) und (2) jeweils bei Nucleotid Nr. 19.

Das auf diese Weise erhaltene DNA-Fragment wurde in einen mit BamHI und HindIII geschnittenen pUC8-Vektor eingesetzt. Das resultierende Plasmid wurde als pUC-D26 bezeichnet.

Ein mit dem Plasmid pUC-D26 transformierter E.coli-Stamm JM109 (Yanisch-Perron et al., Gene 33 (1985), 103) wurde in 100 ml Medium (L-Broth/Ampicillin) über Nacht inkubiert. Am nächsten Morgen wird die Kultur mit 900 ml 2x L-Broth (10 g Trypton, 10 g Hefeextrakt, 5 g NaCl pro Liter)/Ampicillin in einem 3 l-Kolben verdünnt. Nach Zugabe von 2 ml Glycerin und 1 bis 2 Tropfen Silicon-Antischaumemulsion (Fa. Serva) wurde die Kultur dann bei ca. 185 rpm und 37°C für 2 h geschüttelt. Die Induktion und Antigenproduktion erfolgte durch Zugabe von 2 mmol/l des Induktors Isopropylthio-β-D-glactosid (IPTG) und weiteres Schütteln für 3 bis 4 h. Anschließend wurden die Bakterien durch Zentrifugation pelletiert und weiterverarbeitet.

Die Bakterienpellets von zwei 1 l-Kulturen wurden in 200 ml 50 mmol/l Tris-HCl, pH 8,5, 0,2 mg/ml Lysozym und 2 mmol/l Dithioerythritol (DTE) resuspendiert. Anschließend werden EDTA (Endkonzentration: 15 mmol/l), Phenylmethylsulfonylfluorid (Endkonzentration: 1 mmol/l) und 4 mg DNase zugegeben. Die Suspension wurde einige Minuten mit einem Magnetrührer durchmischt und für 45 min bei 37°C in einem Wasserbad inkubiert.

Anschließend erfolgte eine Zugabe von Triton-X100 (Endkonzentration: 1 %) und 30 minütiges Rühren im Magnetrührer. Nach Einfrieren bei -20°C über Nacht und Auftauen wurden die Zellen mindestens 1 Stunde bei 37°C gerührt und gegebenenfalls sonifiziert. Die Inkubation bei 37°C und/oder die Ultraschallbehandlung sollten so lange erfolgen, bis die Viskosität der Suspension mit den lysierten Zellen eindeutig abgenommen hat.

Anschließend wurde bei 35000 g und 4°C für 20 Minuten zentrifugiert. Das resultierende Pellet wurde in 30 ml 50 mmol/l Tris-HCl, pH 8,5, 2 mmol/l DTE, 150 mmol/l EDTA und 1,5 % OGP (Octyl-β-D-glucoapyranosid, Fa. Biomol) resuspendiert. Diese Suspension wurde bei Raumtemperatur auf dem Magnetrührer intensiv für mindestens 3 Stunden gerührt und anschließend bei 35000 g und 4°C für 20 Minuten zentrifugiert.

Das Pellet wurde in 100 ml 8 mol/l Harnstoff, 20 mmol/l Tris-HCl, pH 8,5, 2 mmol/l DTE aufgelöst und gerührt. Das nun gelöste Antigen kann bis zur Weiterverarbeitung bei -20°C eingefroren werden.

Die Aufreinigung des Proteins erfolgte durch die nachfolgend beschriebenen Chromatographieschritte, die bei Raumtemperatur durchgeführt werden. Die Lagerung des Antigens zwischen den Chromatographieschritten erfolgt jeweils bei -20°C.

Der erste Chromatographieschritt erfolgte an einer Q-Sepharose Fast Flow Säule (Pharmacia) mit einem 20 mmol/l Tris-HCl pH 8,5, 8 mol/l Harnstoff, 2 mmol/l DTE-Puffer. Die Elution erfolgte mit einem NaCl-Gradienten (0 bis 0,7 mol/l). Der Durchlauf und die Fraktionen wurden durch SDS-PAGE getestet. Das erfindungsgemäße Polypeptid ist im ersten Hauptpeak enthalten. Die positiven Fraktionen wurden vereinigt und über Nacht gegen ein 10-faches Volumen von 4 mol/l Harnstoff, 2 mmol/l DTE, 20 mmol/l Tris-HCl pH 7,3 dialysiert.

Für den zweiten Chromatographieschritt wurde die gleiche Säule verwendet. Der Säulenpuffer war der nach dem ersten Chromatographieschritt verwendete Dialysepuffer. Die Elution erfolgte mit einem NaCl-Gradienten (0 bis 0,5 mol/l) und anschließend mit 1 mol/l NaCl, NaOH pH 13, 4 mol/l Harnstoff.

Die positiven Fraktionen wurden vereinigt und über Nacht gegen den gleichen Puffer wie oben dialysiert.

Schließlich erfolgte eine Chromatographie an einer S-Sepharose Fast Flow (Pharmacia) Säule. Der Puffer und die Elutionsbedingungen waren gleich wie bei dem vorangehenden Chromatographieschritt.

Das Antigen weist im SDS-Polyacrylamidgel eine Größe von ca. 41 kDa auf. Die Ausbeute beträgt ca. 10 mg Antigen pro Liter Kulturmedium.

### BEISPIEL 2

### Expression des erfindungsgemäßen Antigens im Vergleich mit anderen Antigenen aus dem HCV-NS3-Bereich

Es wurden folgende Antigene exprimiert:
a) erfindungsgemäßes Antigen D26 (Aminosäuren 1207 bis 1488)
b) Antigen C33 (Aminosäuren 1192 bis 1457 entsprechend EP-A-0 450 931)
c) Antigen D27 (Aminosäuren 1227 bis 1528)
d) Antigen NS-3 (Aminosäuren 1007-1534)

Die Expression des Antigens NS-3 erfolgte unter Verwendung des hinterlegten Klons NS-3 (DSM 6847). Die Klonierung und Expression der Antigene C33 und D27 erfolgte entsprechend der in Beispiel 1 beschriebenen Methode. Die kodierenden Bereiche der Aminosäuren 1192 bis 1457 für C33 und der Aminosäuren 1227 bis 1528 für D27 wurden mittels PCR ausgehend von dem Plasmid pUC-N3 aus dem Klon NS-3 nach Standardmethoden amplifiziert. Für C33 wurden die Primer (3) und (4) verwendet, deren Nucleotidsequenzen in SEQ ID NO. 5 bzw. SEQ ID NO. 6 angegeben sind. Für D27 wurden die Primer (5) und (6) verwendet, deren Nucleotidsequenzen in SEQ ID NO. 7 bzw. SEQ ID NO. 8 angegeben sind. Der zu HCV homologe Bereich beginnt in den Primern (3) und (5) mit Nucleotid Nr. 19 und in den Primern (4) und (6) mit Nucleotid Nr. 13.

Die amplifizierten DNA-Fragmente wurden nach Behandlung mit den Restriktionsenzymen BamHI und HindIII und anschließender Reinigung durch Agarosegelelektrophorese in den mit BamHI und HindIII geschnittenen Vektor pUC8 inseriert. Die beiden von den pUC-Vektoren exprimierten Antigene zeigen am N-terminalen Ende einen Bereich von 13 nicht HCV-kodierten Fremdaminosäuren (Met-Thr-Met-Ile-Thr-Asn-Ser-Arg-Gly-Ser-Ile-Met-Lys).

Anschließend wurden die Plasmide in E.coli JM109 transformiert. Bei Klonen, die ein für das C33-Antigen kodierendes DNA-Fragment erhalten, wird ein Antigen mit einem Molekulargewicht von ca. 34 kDa (SDS-PAGE) exprimiert. Bei Klonen mit einem DNA-Fragment, das für das Antigen D27 kodiert, wird eine Bande mit 48 kDa gefunden.

Der Anteil am Gesamtprotein beträgt bei C33 ca. 10 % und ist etwa genau so groß wie bei dem erfindungsgemäßen Polypeptid D26. Für D27 wird eine erheblich schwächere Expression im Bereich von 5 % oder weniger gefunden.

Lysate aus D26, D27 und C33 exprimierenden Klonen wurden nebeneinander im SDS-Gel aufgetrennt und auf Nitrocellulose transferiert. Nach Inkubation über Nacht mit verschiedenen HCV-positiven Seren in 1:100 Verdünnung erfolgte ein Nachweis gebundener Antikörper mittels eines Anti-human-IgG-Antikörper-Peroxidase-Konjugats und Farbreaktion mit 3,3'-Diaminobenzidintetrachlorid (DAB)/Wasserstoffperoxid.

Bei stark positiven Seren ist bei allen HCV-Antigensegmenten eine gute Reaktivität zu finden. Im Fall von schwach positiven NS3-HCV-Seren zeigt das C33-Antigen im Vergleich zu dem erfindungsgemäßen Antigen in einigen Fällen die gleiche und in anderen Fällen eine etwas schwächere Reaktivität. Bei D27 findet man jedoch eine deutlich verminderte Farbreaktion mit schwach NS3-positiven Seren.

### BEISPIEL 3

### Untersuchung von Spezifität und Sensitivität von verschiedenen Antigenen aus der NS3-Region von HCV

Mit dem indirekten Testkonzept wurde die Reaktivität verschiedener Antigene aus dem NS3-Bereich mit insgesamt 960 negativen Seren vergleichend bewertet. Bei dieser Bewertung wurde eine deutlich überlegene Spezifität des erfindungsgemäßen Antigens D26 gegenüber dem Vergleichsantigen C33 gefunden, die sich in einer erheblich geringeren Anzahl an falsch positiven Ergebnissen äußert.

| Konstrukt | falsch positive Bewertung | richtig negative Bewertung |
|---|---|---|
| C33 | 8 | 952 |
| D26 | 2 | 958 |

Weiterhin wurde mit dem indirekten Testkonzept die Reaktivität verschiedener NS3-Konstrukte mit 20 Seren mit gesichertem HCV-Status vergleichend bewertet. Bei vergleichbarer Sensitivität der Konstrukte NS3 (Aminosäuren 1007 bis 1534), C33, D26 und D27 wurde eine eine geringere Sensitivität des Antigens D27 gefunden.

| Konstrukt | richtig positive Bewertung | falsch negative Bewertung |
|---|---|---|
| NS3 | 20 | 0 |
| C33 | 20 | 0 |
| D26 | 20 | 0 |
| D27 | 19 | 1 |

### BEISPIEL 4

### Untersuchung der Stabilität von Antigenen aus der NS3-Region von HCV

Mit dem indirekten Testkonzept wurde die Stabilität der Antigene NS3 und D26 vergleichend bewertet. Es wurde eine deutlich schlechtere Stabilität des Antigens NS3 gegenüber dem erfindungsgemäßen Antigen D26 gefunden. Die Stabilität der Antigene wurde nach 72 h Inkubation bei 37°C untersucht.

| Konstrukt | Proben Nr. | Signalwiederfindung- |
|---|---|---|
| NS3 | 1 | < 20 % |
| | 2 | < 20 % |
| D26 | 1 | 99 % |
| | 2 | 103 % |

### BEISPIEL 5

### Untersuchung der Reaktivität des HCV-NS3-Helicase-Antigens in An- bzw. Abwesenheit von reduzierenden Reagenzien

Es wurde der Zeitpunkt der Serokonversion anhand der Reaktivität von zu verschiedenen Zeitpunkten entnommenen Serumproben mit HCV-NS3-Helicase-Antigen unter physiologischen Bedingungen mit bzw. ohne 20 mmol/l DTT getestet. Das Testkonzept war ein Doppelantigen-Brückentest zur klassenunabhängigen Erkennung aller Immunglobuline, bei dem ein mit einer elektrochemischen Markierungsgruppe (Ruthenium-Metallkomplex) versehenes und ein an eine Festphase bindefähiges Antigen (biotinyliertes Antigen) verwendet wurde.

Das Ergebnis dieses Tests ist in der nachstehenden Tabelle gezeigt. Es ist zu erkennen, daß in Anwesenheit von 20 mmol/l DTT eine um 38 Tage frühere Erkennung der Serokonversion möglich war. Weiterhin findet man in Anwesenheit von DTT eine verbesserte Signalstärke.

**TABELLE**

| Tag der Blutentnahme | Tage nach Beginn der Blutentnahme | Reaktivität des NS3-Antigens ohne DTT (Signal/Cut off) | Reaktivität des NS3-Antigens mit DTT (Signal/ Cut off) |
|---|---|---|---|
| 28.07.1988 | 0 | 0.1 | 0.1 |
| 01.08.1988 | 4 | 0.1 | 0.1 |
| 08.08.1988 | 11 | 0.1 | 0.1 |
| 11.08.1988 | 14 | 0.1 | 0.1 |
| 15.08.1988 | 18 | 0.1 | 0.1 |
| 25.08.1988 | 28 | 0.1 | 0.1 |
| 29.08.1988 | 32 | 0.1 | 1.6* |
| 14.09.1988 | 48 | 0.1 | 6.5* |
| 05.10.1988 | 69 | 1.3* | 4.8* |
| 19.10.1988 | 83 | 2.1* | 6.8* |

| | | | |
|---|---|---|---|
| * positives Signal | | | |

## Patentansprüche

1. Polypeptid, das aus den Aminosäuren 1207 ± 10 bis 1488 ± 10 eines Hepatitis C Virus und weniger als 20 fremden Aminosäuren besteht.

2. Polypeptid nach Anspruch 1, das die Aminosäuren 1207 ± 5 bis 1488 ± 5 eines Hepatitis C Virus enthält.

3. Polypeptid nach Anspruch 1 oder 2, das die Aminosäuren 1207 ± 2 bis 1488 ± 2 eines Hepatitis C Virus enthält.

4. Polypeptid nach einem der Ansprüche 1 bis 3, das die Aminosäuren 1207 bis 1488 eines Hepatitis C Virus enthält.

5. Polypeptid nach einem der Ansprüche 1 bis 4, das die Aminosäuren 14 - 295 der in SEQ ID NO. 1 dargestellten Aminosäuresequenz oder eine dazu mindestens 90% homologe Aminosäuresequenz enthält.

6. Polypeptid nach einem der Ansprüche 1 bis 5, das mindestens eine Markierungsgruppe trägt.

7. Polypeptid nach einem der Ansprüche 1 bis 6,
**dadurch gekennzeichnet**,
daß es eine oder mehrere Sulfhydrylgruppen in kovalent modifizierter Form enthält.

8. Polypeptid nach einem der Ansprüche 1 bis 6,
**dadurch gekennzeichnet**,
daß ein oder mehrere Cysteinreste durch andere Aminosäuren ersetzt sind.

9. Nucleinsäure,
**dadurch gekennzeichnet**,
daß sie für ein Polypeptid nach einem der Ansprüche 1 bis 8 kodiert.

10. Nucleinsäure nach Anspruch 9,
**dadurch gekennzeichnet**,
daß sie
(a) die Nucleotide 40 - 885 der in SEQ ID NO. 1 dargestellten Nucleotidsequenz oder
(b) eine der Sequenz aus (a) im Rahmen der Degeneration des genetischen Codes entsprechende Nucleotidsequenz enthält.

11. Vektor,
**dadurch gekennzeichnet**,
daß er mindestens eine Kopie einer Nucleinsäure nach Anspruch 9 oder 10 enthält.

12. Vektor pUC-D26.

13. Zelle,
**dadurch gekennzeichnet**,
daß sie mit mindestens einer Kopie einer Nucleinsäure nach Anspruch 9 oder 10 oder eines Vektors nach Anspruch 11 oder 12 transformiert ist.

14. Verwendung eines Polypeptids nach einem der Ansprüche 1 bis 8 als Antigen in einem immunologischen Testverfahren oder als Helicase-Protein.

15. Verfahren zur immunologischen Bestimmung eines gegen Hepatitis C Virus gerichteten Antikörpers in einer Probeflüssigkeit,
**dadurch gekennzeichnet**,
daß man die Probeflüssigkeit mit mindestens einem Polypeptid nach einem der Ansprüche 1 bis 8 inkubiert und den Antikörper über eine Bindung mit dem Polypeptid nachweist.

16. Verfahren nach Anspruch 15,
**dadurch gekennzeichnet**,
daß man die Probeflüssigkeit mit zwei Polypeptiden P₁ und P₂ inkubiert, wobei das Polypeptid P₁ (a) an eine Festphase gebunden ist oder (b) in einer an eine Festphase bindefähigen Form vorliegt und das Polypeptid P₂ eine Markierungsgruppe trägt, und den Antikörper durch Bestimmung der Markierung in der Festphase oder/und in der flüssigen Phase nachweist.

17. Verfahren nach Anspruch 15,
**dadurch gekennzeichnet**,
daß man die Probeflüssigkeit mit einem Polypeptid P₁, das (a) an eine Festphase gebunden ist oder (b) in einer an eine Festphase bindefähigen Form vorliegt und mit einem weiteren, gegen P₁ gerichteten Antikörper, der eine Markierungsgruppe trägt, inkubiert und den zu bestimmenden Antikörper durch Bestimmung der Markierung in der Festphase oder/und in der flüssigen Phase nachweist.

18. Verfahren nach einem der Ansprüche 15 bis 17,
**dadurch gekennzeichnet**,
daß man die Bestimmung des Antikörpers unter reduzierenden Bedingungen durchführt.

19. Reagenz zur immunologischen Bestimmung eines gegen Hepatitis C Virus gerichteten Antikörpers,
**dadurch gekennzeichnet**,
daß es mindestens ein Polypeptid nach einem der Ansprüche 1 bis 8 enthält.

20. Verwendung eines Polypeptids nach einem der Ansprüche 1 bis 8 zur Herstellung eines Impfstoffs.

21. Verfahren zur immunologischen Bestimmung eines gegen Hepatitis C Virus gerichteten Antikörpers in einer Probeflüssigkeit, wobei man die Probeflüssigkeit mit mindestens einem Polypeptid, das Sequenzbereiche aus Hepatitis C Virus, insbesondere aus der NS3-Region von Hepatitis C Virus enthält, inkubiert und den Antikörper über eine Bindung mit dem Polypeptid nachweist,
**dadurch gekennzeichnet**,
daß man ein Polypeptid aus einem Bereich, der mindestens einen Cysteinrest enthält, verwendet und (a) die Bestimmung des Antikörpers unter reduzierenden Bedingungen durchführt, (b) einen oder mehrere Cysteinreste kovalent modifiziert oder/und (c) einen oder mehrere Cysteinreste durch andere Aminosäuren ersetzt.


SEQUENZPROTOKOLL

(1) ALGEMEINE INFORMATION:

(i) ANMELDER:
(A) NAME: Boehringer Mannheim GmbH
(B) STRASSE: Sandhofer Str. 112-132
(C) ORT: Mannheim
(E) LAND: Deutschland
(F) POSTLEITZAHL: 68305

(ii) ANMELDETITEL: Rekombinantes Antigen aus der NS3-Region des
Hepatitis C Virus

(iii) ANZAHL DER SEQUENZEN: 8

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:
(A) LÄNGE: 885 Basenpaare
(B) ART: Nukleinsäure
(C) STRANGFORM: beides
(D) TOPOLOGIE: linear

(ii) ART DES MOLEKÜLS: cDNS

(vi) URSPRÜNLICHE HERKUNFT:
(A) ORGANISMUS: Hepatitis C Virus

(viii) POSITION IM GENOM:
(A) CHROMOSOM/SEGMENT: NS3

(ix) MERKMALE:
(A) NAME/SCHLÜSSEL: CDS
(B) LAGE: 1..885

(xi) SEQUENZBESCHREIBUNG: SEQ ID NO: 1:

ATG ACC ATG ATT ACG AAT TCC CGG GGA TCC ATC ATG AAA TCC CCG GTG 48
Met Thr Met Ile Thr Asn Ser Arg Gly Ser Ile Met Lys Ser Pro Val
1               5                   10                  15

TTC ACG GAT AAC TCC TCT CCA CCG GTA GTG CCC CAG AGC TTC CAG GTG 96
Phe Thr Asp Asn Ser Ser Pro Pro Val Val Pro Gln Ser Phe Gln Val
            20                  25                  30

GCT CAC CTG CAT GCT CCC ACA GGC AGC GGC AAG AGC ACC AAG GTC CCG 144
Ala His Leu His Ala Pro Thr Gly Ser Gly Lys Ser Thr Lys Val Pro
        35                  40                  45

GCT GCA TAC GCA GCT CAG GGC TAC AAG GTG CTA GTG CTC AAC CCT TCT 192
Ala Ala Tyr Ala Ala Gln Gly Tyr Lys Val Leu Val Leu Asn Pro Ser
    50                  55                  60

GTT GCT GCA ACA TTG GGC TTT GGT GCC TAC ATG TCC AAG GCT CAT GGG 240
Val Ala Ala Thr Leu Gly Phe Gly Ala Tyr Met Ser Lys Ala His Gly
65                  70                  75                  80

ATC GAT CCT AAC ATC AGG ACC GGG GTG AGA ACA ATT ACC ACT GGC AGC 288

Ile Asp Pro Asn Ile Arg Thr Gly Val Arg Thr Ile Thr Thr Gly Ser
                85                  90                  95

CCC ATT ACG TAC TCC ACT TAC GGC AAG TTT CTT GCC GAC GGC GGG TGC     336
Pro Ile Thr Tyr Ser Thr Tyr Gly Lys Phe Leu Ala Asp Gly Gly Cys
            100                 105                 110

GCA GGG GGT GCT TAT GAC ATA ATA ATT TGT GAC GAG TGC CAC TCC ACG     384
Ala Gly Gly Ala Tyr Asp Ile Ile Ile Cys Asp Glu Cys His Ser Thr
        115                 120                 125

GAT GCC ACA TCC ATC TTG GGC ATC GGC ACT GTC CTT GAC CAA GGA GAG     432
Asp Ala Thr Ser Ile Leu Gly Ile Gly Thr Val Leu Asp Gln Gly Glu
    130                 135                 140

ACT GCG GGG GCG AAA TTG GTT GTG TTC GCC ACC GCC ACC CCT CCG GGC     480
Thr Ala Gly Ala Lys Leu Val Val Phe Ala Thr Ala Thr Pro Pro Gly
145                 150                 155                 160

TCC GTC ACT GTG CCC CAT CCC AAC ATT GAG GAG GTT GCT CTA TCC ACC     528
Ser Val Thr Val Pro His Pro Asn Ile Glu Glu Val Ala Leu Ser Thr
                165                 170                 175

ACC GGA GAG ATC CCT TTT TAC GGC AAG GCT ATC CCC CTT GAG GTA ATC     576
Thr Gly Glu Ile Pro Phe Tyr Gly Lys Ala Ile Pro Leu Glu Val Ile
            180                 185                 190

AAG GGG GGG AGA CAT CTC ATC TTC TGT CAT TCA AAG AGG AAG TGC GAT     624
Lys Gly Gly Arg His Leu Ile Phe Cys His Ser Lys Arg Lys Cys Asp
        195                 200                 205

GAG CTC GCC ACA AAG CTG GTC GCA ATG GGC ATC AAT GCC GTG GCC TAC     672
Glu Leu Ala Thr Lys Leu Val Ala Met Gly Ile Asn Ala Val Ala Tyr
    210                 215                 220

TAC CGC GGT CTT GAC GTG TCC GTC ATC CCG ACC AGC GGT GAT GTT GTC     720
Tyr Arg Gly Leu Asp Val Ser Val Ile Pro Thr Ser Gly Asp Val Val
225                 230                 235                 240

GTC GTG GCA ACC GAC GCC CTC ATG ACC GGC TAT ACC GGC GAC TTC GAC     768
Val Val Ala Thr Asp Ala Leu Met Thr Gly Tyr Thr Gly Asp Phe Asp
                245                 250                 255

TCG GTG ATA GAC TGC AAC ACG TGT GTC ACT CAG ACA GTC GAT TTC AGC     816
Ser Val Ile Asp Cys Asn Thr Cys Val Thr Gln Thr Val Asp Phe Ser
            260                 265                 270

CTT GAC CCT ACC TTC ACC ATT GAG ACG ACC ACA CTT CCC CAG GAT GCT     864
Leu Asp Pro Thr Phe Thr Ile Glu Thr Thr Thr Leu Pro Gln Asp Ala
        275                 280                 285

GTC TCC CGC ACT CAA CGA CGG                                         885
Val Ser Arg Thr Gln Arg Arg
    290                 295

(2) INFORMATION ZU SEQ ID NO: 2:

     (i) SEQUENZ CHARAKTERISTIKA:
        (A) LÄNGE: 295 Aminosäuren
        (B) ART: Aminosäure
        (D) TOPOLOGIE: linear

    (ii) ART DES MOLEKÜLS: Protein

    (xi) SEQUENZBESCHREIBUNG: SEQ ID NO: 2:

Met Thr Met Ile Thr Asn Ser Arg Gly Ser Ile Met Lys Ser Pro Val

1                   5                   10                  15

Phe Thr Asp Asn Ser Ser Pro Pro Val Val Pro Gln Ser Phe Gln Val
                20                  25                  30

Ala His Leu His Ala Pro Thr Gly Ser Gly Lys Ser Thr Lys Val Pro
            35                  40                  45

Ala Ala Tyr Ala Ala Gln Gly Tyr Lys Val Leu Val Leu Asn Pro Ser
    50                  55                  60

Val Ala Ala Thr Leu Gly Phe Gly Ala Tyr Met Ser Lys Ala His Gly
65                  70                  75                  80

Ile Asp Pro Asn Ile Arg Thr Gly Val Arg Thr Ile Thr Thr Gly Ser
                85                  90                  95

Pro Ile Thr Tyr Ser Thr Tyr Gly Lys Phe Leu Ala Asp Gly Gly Cys
            100                 105                 110

Ala Gly Gly Ala Tyr Asp Ile Ile Ile Cys Asp Glu Cys His Ser Thr
        115                 120                 125

Asp Ala Thr Ser Ile Leu Gly Ile Gly Thr Val Leu Asp Gln Gly Glu
    130                 135                 140

Thr Ala Gly Ala Lys Leu Val Val Phe Ala Thr Ala Thr Pro Pro Gly
145                 150                 155                 160

Ser Val Thr Val Pro His Pro Asn Ile Glu Glu Val Ala Leu Ser Thr
                165                 170                 175

Thr Gly Glu Ile Pro Phe Tyr Gly Lys Ala Ile Pro Leu Glu Val Ile
            180                 185                 190

Lys Gly Gly Arg His Leu Ile Phe Cys His Ser Lys Arg Lys Cys Asp
        195                 200                 205

Glu Leu Ala Thr Lys Leu Val Ala Met Gly Ile Asn Ala Val Ala Tyr
    210                 215                 220

Tyr Arg Gly Leu Asp Val Ser Val Ile Pro Thr Ser Gly Asp Val Val
225                 230                 235                 240

Val Val Ala Thr Asp Ala Leu Met Thr Gly Tyr Thr Gly Asp Phe Asp
                245                 250                 255

Ser Val Ile Asp Cys Asn Thr Cys Val Thr Gln Thr Val Asp Phe Ser
            260                 265                 270

Leu Asp Pro Thr Phe Thr Ile Glu Thr Thr Thr Leu Pro Gln Asp Ala
        275                 280                 285

Val Ser Arg Thr Gln Arg Arg
    290                 295

(2) INFORMATION ZU SEQ ID NO: 3:

    (i) SEQUENZ CHARAKTERISTIKA:
        (A) LÄNGE: 40 Basenpaare
        (B) ART: Nukleinsäure
        (C) STRANGFORM: Einzel
        (D) TOPOLOGIE: linear

    (ii) ART DES MOLEKÜLS: cDNS

(xi) SEQUENZBESCHREIBUNG: SEQ ID NO: 3:

AAGGGATCCA TCATGAAATC CCCGGTGTTC ACGGATAACT 40

(2) INFORMATION ZU SEQ ID NO: 4:

(i) SEQUENZ CHARAKTERISTIKA:
(A) LÄNGE: 39 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 4:

GGGAAGCCTT AATTCTTACC GTCGTTGAGT GCGGGAGAC 39

(2) INFORMATION ZU SEQ ID NO: 5:

(i) SEQUENZ CHARAKTERISTIKA:
(A) LÄNGE: 39 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 5:

GAGGGATCCA TCATGAAAGC GGTGGACTTT ATCCCTGTG 39

(2) INFORMATION ZU SEQ ID NO: 6:

(i) SEQUENZ CHARAKTERISTIKA:
(A) LÄNGE: 33 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 6:

GAGAAGCTTT TAACACGTGT TGCAGTCTAT CAC 33

(2) INFORMATION ZU SEQ ID NO: 7:

(i) SEQUENZ CHARAKTERISTIKA:
(A) LÄNGE: 39 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 7:

GAGGGATCCA TCATGAAACA CCTGCATGCT CCCACCGGC 39

(2) INFORMATION ZU SEQ ID NO: 8:

(i) SEQUENZ CHARAKTERISTIKA:
(A) LÄNGE: 33 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 8:

GAGAAGCTTT TAATACCAAG CACAGCCTGC GTC 33